# EUROPEAN PATENT SPECIFICATION

(11) **EP 3 351 122 B1**
(45) Date of publication and mention of the grant of the patent: **17.06.2020**
(21) Application number: 17779986.3
(22) Date of filing: 14.08.2017
(51) Int. Cl.: A24F 47/00, A24F 7/00

(54) **MOUTH PIECE OF ELECTRONIC CIGARETTE, E-LIQUID CARTRIDGE, AND ELECTRONIC CIGARETTE**
MUNDSTÜCK FÜR ELEKTRONISCHE ZIGARETTE, FLÜSSIGKEITSKARTUSCHE FÜR ELEKTRONISCHE ZIGARETTE UND ELEKTRONISCHE ZIGARETTE
EMBOUT DE CIGARETTE ÉLECTRONIQUE, CARTOUCHE DE LIQUIDE À VAPOTER ET CIGARETTE ÉLECTRONIQUE

(30) Priority: 12.10.2016 CN 201621116845 U
(43) Date of publication of application: 25.07.2018
(73) Proprietor: Changzhou Patent Electronic Technology Co., Ltd, Jiangsu 213001 (CN)
(72) Inventor: QIU, Weihua, Jiangsu 213125 (CN)
(74) Representative: Sun, Yiming
(86) International application number: PCT/CN2017/097396
(87) International publication number: WO 2018/068577

(56) References cited:
- WO-A1-2014/130176
- CN-U- 201 839 801
- CN-U- 201 839 801
- CN-U- 202 154 026
- CN-U- 202 154 026
- CN-U- 203 087 529
- CN-U- 203 341 011
- CN-U- 203 608 850
- US-A- 2 065 223
- US-A1- 2015 013 700
- US-A1- 2015 335 074

## Description

### FIELD

The present disclosure relates to the technical field of electronic cigarettes, and more particularly, to a cigarette mouthpiece, a cartomizer and an electronic cigarette therefor.

In particular the invention relates to a cigarette mouthpiece for an electronic cigarette comprising a hard substrate having a top end and a bottom end, and a soft substrate, a through hole being defined inside the hard substrate and extending through the opposite ends of the hard substrate, the through hole being configured as a smoke passage, an opening of the through hole located in the top end of the through hole being configured as a smoke outlet, the soft substrate being mounted on the outside of the hard substrate and supported by the hard substrate, wherein a concave portion is located between the top end and the bottom end, a groove is defined on the concave portion, and the soft substrate is mounted in the groove.

The invention also relates to a cartomizer comprising a main body and such cigarette mouthpiece, and an electronic cigarette comprising such mouthpiece.

### BACKGROUND

US 2,065,223 A discloses a mouthpiece of the generic type as defined above.

In particular US 2,065,223 A discloses a stem for smoking pipes and like articles, the stem having a mouthpiece provided in a portion thereof with a recess having notches therein, and a bit disposed in the recess and provided with projections engaging in the notches.

US 2015/0335074 A1 discloses a handheld vaporizing device with improved heating element and temperature control, improved e-liquid reservoir, and capacitive technology to activate the heating element to prime the device to deliver aerosol on demand.

CN 202 154 026 U discloses a cigarette holder comprising a cigarette holder body, a filter and a sleeve, wherein the sleeve comprises a filtration cavity, a cigarette insertion part and a smoke channel. The filter is arranged on the cigarette holder body, the cigarette holder body is formed through plastic injection, the filtration cavity of the sleeve is sleeved outside the filter and is connected with the cigarette holder body, and a rubber mouthpiece is arranged on the cigarette holder body.

US 2015/0013700 A1 discloses an electronic cigarette including a mouthpiece, a bracket for the mouthpiece, an atomization pole including a threaded copper ring, a silicate ring, a heating wire, a joint, an insulating ring, a filter cotton, and a fluid stop ring. The mouthpiece is made of silica gel and is connected with the atomization pole via the bracket. The heating wire is electrically welded using nickel-chrome as a material. The heating wire is spiral. One end of the threaded copper ring is connected with the joint. The insulating ring is disposed between the thread ring and the joint. The silicate ring is disposed between the filter cotton and the threaded copper ring. The fluid stop ring is disposed between the bracket and the heating wire for preventing the leakage of fluid.

CN 201 839 801 U discloses an electronic cigarette and a cigarette cartridge thereof. The electronic cigarette and the cigarette cartridge thereof comprise casings, wherein the casings comprise a soft cigarette holder with a sucking hole, or soft layers are partially or completely arranged on the surfaces of the casings.

Presently, cigarette mouthpieces for electronic cigarettes are usually made of hard plastic or soft materials. A cigarette mouthpiece made of soft materials can provide a better tactility and have a wider application. However, the connection of the cigarette mouthpiece made of soft material is not reliable. And the service life of the cigarette mouthpiece made of soft material is shorter than that of the cigarette mouthpiece made of hard plastic. The cigarette mouthpiece made of soft material may discolor and be contaminated easily.

### SUMMARY

It is an object of the present disclosure to solve the defects in the prior art by providing a cigarette mouthpiece which has a simple structure and is configured to improve the user's tactility, and to provide a cartomizer and an electronic cigarette therefor.

A cigarette mouthpiece for an electronic cigarette of the present disclosure is as follows:
The cigarette mouthpiece for an electronic cigarette comprises a hard substrate having a top end and a bottom end, and a soft substrate, a through hole being defined inside the hard substrate and extending through the opposite ends of the hard substrate, the through hole being configured as a smoke passage, an opening of the through hole located in the top end of the through hole being configured as a smoke outlet, the soft substrate being mounted on the outside of the hard substrate and supported by the hard substrate, wherein a concave portion is located between the top end and the bottom end, a groove is defined on the concave portion, and the soft substrate is mounted in the groove. In addition both the number of the groove and the soft substrate are two, the two grooves are respectively defined on the opposite sides of the hard substrate, and the two soft substrates are respectively mounted in the corresponding grooves.

In one embodiment, the soft substrate is made of one of a group of silicon resin, latex and TPE.

In one embodiment, the soft substrate is disposed around the hard substrate.

In one embodiment, the soft substrate has a shape selected from one of a group of circular, square and ellipse.

In one embodiment, the top end is relatively narrower than the bottom end. When the soft substrate is mounted in the groove, the outer surface of the entire concave portion becomes a curved surface accordingly.

In one embodiment, a retaining hole is defined on the soft substrate. A retaining protrusion corresponding to the retaining hole is defined on the concave portion and disposed to insert into the retaining hole.

In one embodiment, the soft substrate is made of one of a group of silicon resin, latex, EVA, silicon rubber and TPE.

In one embodiment, the soft substrate is disposed around the hard substrate.

In one embodiment, the hard substrate comprises an upper section and a lower section, wherein the diameter of the upper section is smaller than that of the lower section. The groove in an annular shape is defined on the outside of the upper section along the circumferential direction of the upper section. Accordingly, the soft substrate is arranged along the circumferential direction of the upper section.

In one embodiment, the hard substrate comprises a top end and a bottom end. A concave portion is located between the top end and the bottom end. A groove is defined on the concave portion, and the soft substrate is mounted in the groove.

In one embodiment, both the number of the groove and the soft substrate are two. The two grooves are respectively defined on the opposite sides of the hard substrate. The two soft substrates are respectively mounted in the corresponding grooves.

In one embodiment, the soft substrate is a sheet, and the two ends of the soft substrate are relatively thinner than the middle portion of the soft substrate.

In one embodiment, the soft substrate is melted at a high temperature and then adhered to the groove.

In one embodiment, the soft substrate is disposed around the hard substrate.

In one embodiment, a retaining protrusion is defined on the concave portion, and a retaining hole is defined on the soft substrate. The soft substrate is mounted on the outside of the hard substrate through the cooperation of the retaining protrusion and the retaining hole. Alternatively, two retaining protrusions are defined on the hard substrate along the circumferential direction of the hard substrate. One retaining protrusion is adjacent to the top end, and the other retaining protrusion is adjacent to the bottom end. The soft substrate is mounted between the two retaining protrusions.

In one embodiment, the hard substrate comprises an upper section and a lower section. A groove is defined on the outside of the upper section along the circumferential direction of the upper section. The soft substrate is mounted in the groove along the circumferential direction of the upper section.

In one embodiment, the hard substrate is made of one of a group of hard plastic, stainless steel and glass.

In one embodiment, the soft substrate is made of one of a group of silicon resin, latex, TPE, EVA and soft silicone rubber.

The present invention also provides a cartomizer which comprises a main body and a cigarette mouthpiece mounted on the main body. The cigarette mouthpiece is any one of the above described cigarette mouthpiece.

In one embodiment, the cigarette mouthpiece is detachably connected to the main body.

In one embodiment, the cigarette mouthpiece is fixedly connected to the main body.

The present invention further provides an electronic cigarette having a cartomizer. The cartomizer is any one of the above described cartomizer.

The present invention additionally provides an electronic cigarette having a cigarette mouthpiece. The cigarette mouthpiece is any one of the above described cigarette mouthpieces.

The beneficial effects of the present disclosure are as follows:
When the user's lips come in contact with the cigarette mouthpiece, the user can obtain soft and comfortable tactility through the soft substrate mounted on the hard substrate. At the same time, the use of the hard substrate in cooperation with the soft substrate ensures the reliability of the cigarette mouthpiece, reduces the risk of contamination and prolongs the service life.

Both the front side and the rear side of the flat cigarette mouthpiece have a groove. Two soft substrates are mounted in the corresponding grooves, respectively. The soft substrates are only mounted on the place which can be contacted by the user's lips such that materials can be saved and process can be simplified.

The soft substrate is disposed around the cylindrical cigarette mouthpiece such that there is no need to adjust the angle of the cigarette mouthpiece to obtain good tactility. Therefore, it is more convenient for the user's operation.

### BRIEF DESCRIPTION OF THE DRAWINGS

The drawings are provided for further understanding of the disclosure and constitute a part of the specification, and are used, together with the following detailed description, to explain the disclosure, and should not be construed as a limitation for the disclosure. In the drawings,
FIG. 1 is a perspective schematic view of a cigarette mouthpiece according to an embodiment of the present disclosure.
FIG. 2 is a side view of the cigarette mouthpiece shown in FIG. 1.
FIG. 3 is an exploded schematic view of the cigarette mouthpiece shown in FIG. 1.
FIG. 4 is a perspective schematic view of a cartomizer comprising the cigarette mouthpiece shown in FIG. 1.
FIG. 5 is a perspective schematic view of a cigarette mouthpiece according to another embodiment of the present disclosure.
FIG. 6 is a side view of the cigarette mouthpiece shown in FIG. 5.
FIG. 7 is a perspective schematic view of a cigarette mouthpiece according to a still another embodiment of the present disclosure.
FIG. 8 is a perspective schematic view of an atomizer comprising the cigarette mouthpiece shown in FIG. 7.
FIG. 9 is a cross-sectional view of the cigarette mouthpiece shown in FIG.7.

### NAME AND REFERENCE NUMERAL OF COMPONENTS

| | | |
|---|---|---|
| Hard Substrate 10 | Top End 11 | Bottom End 12 |
| Retaining Protrusion 13 | Groove 14 | Upper Section 15 |
| Lower Section 16 | Smoke Outlet 17 | Concave Portion 18 |
| Latching Structure 19 | Soft Substrate 20 | Retaining Hole 21 |
| Main Body 31 | | |

### DETAILED DESCRIPTION

Specific embodiments of the present disclosure will be described in detail below with reference to the drawings. It should be understood that the specific embodiments described herein are for the purpose of illustration and explanation of the present disclosure, and are not intended to limit the disclosure.

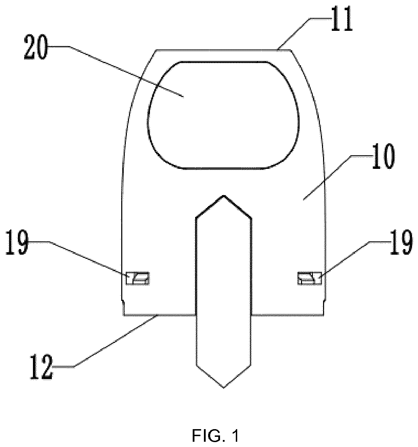

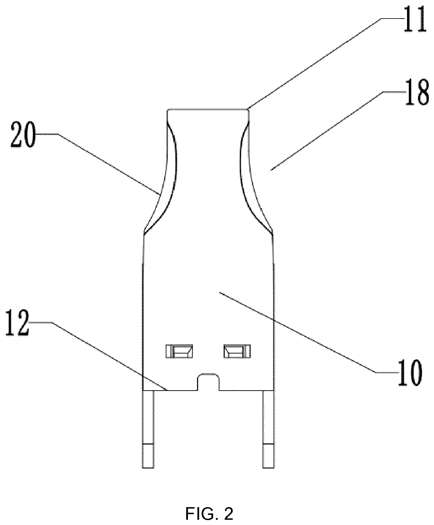

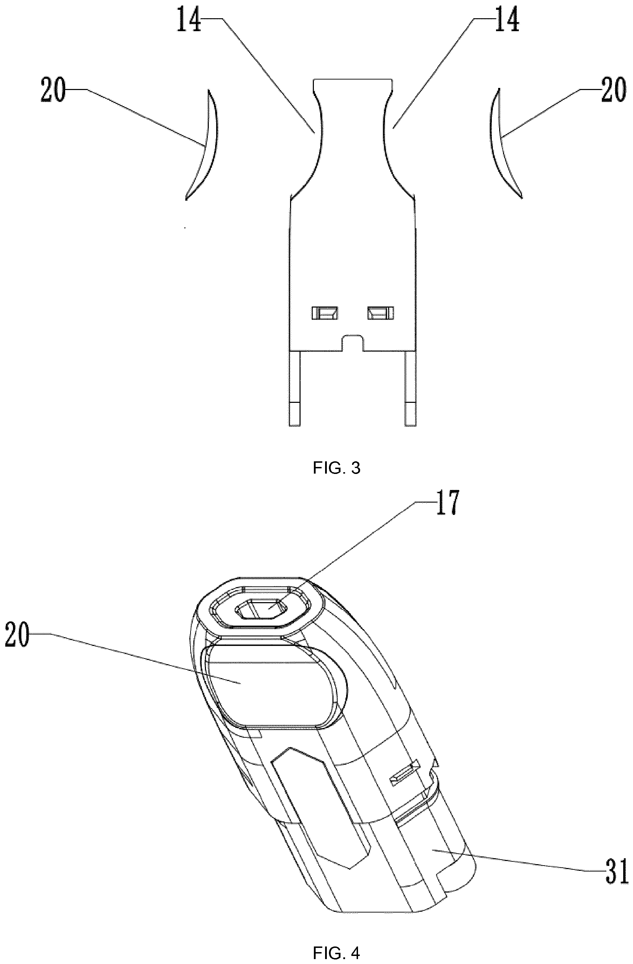

As shown in FIGs.1-4, a cigarette mouthpiece for an electronic cigarette is provided in the present disclosure. The cigarette mouthpiece comprises a hard substrate 10. A soft substrate 20 is mounted on the outside of the hard substrate 10. In one embodiment, the hard substrate 10 is made of one of a group of hard plastic, stainless steel and glass. It can be appreciated that the hard substrate 10 can be made of other hard materials to support the soft substrate 20. A through hole (not shown) is defined inside the hard substrate 10 and extends through its upper end and lower end. The through hole is mainly configured as a smoke passage when the user inhales. It can be appreciated that if the electronic cigarette is an automatic electronic cigarette, an independent inductive passage can be defined inside the through hole.

In one embodiment, the hard substrate 10 having an approximately flat shape comprises a relatively narrower top end 11 and a relatively wider bottom end 12. The bottom end 12 is configured to connect to a main body 31 of a cartomizer. The bottom end 12 can have any shape, such as a rectangular parallelepiped shape or a cylindrical shape. In one embodiment, the bottom end 12 is detachably connected to the main body 31 by clamping or screwing. It can be appreciated that the fixed connection, such as snap fit connection, is also applicable. In this embodiment, the cigarette mouthpiece and the main body 31 are fixed together via a latching structure 19. In this embodiment, the top end 11 has a rectangular parallelepiped shape. It can be appreciated that, the top end 11 can have any shape, such as a cylindrical shape or an ellipse cylinder shape.

A concave portion 18 is located between the top end 11 and the bottom end 12. In one embodiment, there are two concave portions 18. The two concave portions 18 are located on the front side and the rear side of the hard substrate 18, respectively. In one embodiment, each concave portion 18 defines a groove 14. When the soft substrate 20 is mounted in the groove 14, the outer surface of the entire concave portion 18 becomes a smooth curved surface accordingly.

A through hole is defined inside the hard substrate 10 and extends through the top end 11 and the bottom end 12. Smoke outlet 17 is an opening of the through hole located at the top end 11.

When the user inhales, the smoke can be sucked off from the smoke outlet 17. The lips of the user contact with the soft substrate 20, and the user can obtain comfortable tactility.

The soft substrate 20 can be made of elastic materials. The appropriate elastic material can be selected from one of a group of silicone resin, latex, thermoplastic elastomer (TPE), ethylene-vinyl acetate copolymer (EVA) and soft silicone rubber. It can be appreciated that other soft and environmentally friendly materials are also applicable.

Both of the opposite sides of the hard substrate 10 define a groove 14, and the two soft substrates 20 are mounted in the corresponding grooves 14 respectively.

In one embodiment, the soft substrate 20 is a sheet. Specifically, the soft substrate 20 has a shape selected from one of a group of circular, square and ellipse. Furthermore, the two ends of the soft substrate 20 are relatively thinner than the middle portion of the soft substrate 20. The shape of the groove 14 is configured to fit such soft substrate 20.

In one embodiment, the soft substrate 20 is melted at a high temperature and then adhered to the groove 14.

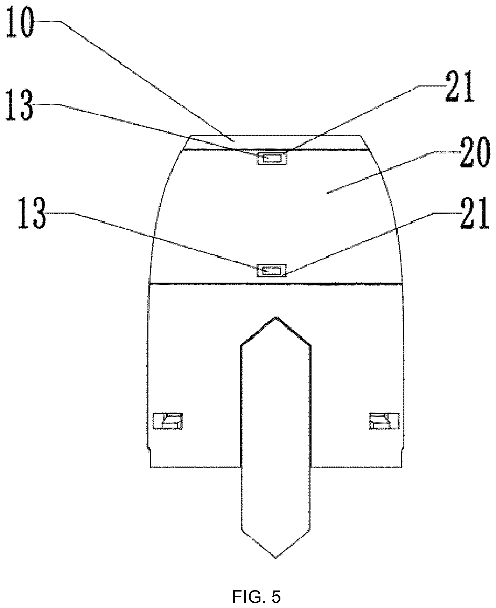

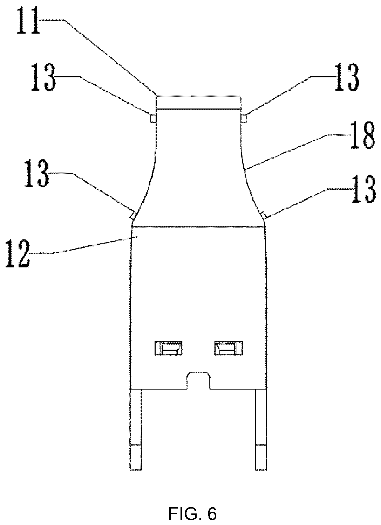

As shown in FIG.5 and FIG.6, in one embodiment, the soft substrate 20 is disposed around the hard substrate 10. That is, the soft substrate 20 has a sleeve shape and is sleeved on the outside of the hard substrate 10. In one embodiment, a retaining protrusion 13 is defined on the concave portion 18, and a retaining hole 21 corresponding to the retaining protrusion 13 is defined on the soft substrate 20. The retaining protrusion 13 inserts into the retaining hole 21 to prevent the soft substrate 20 from slipping.

It can be appreciated that two retaining protrusions 13 configured to retain the soft substrate 20 can be defined on the outside of the hard substrate 10 along the circumferential direction of the hard substrate 10. One retaining protrusion 13 is adjacent to the top end 11, and the other retaining protrusion 13 is adjacent to the bottom end 12. The soft substrate 20 is located between the two retaining protrusions 13.

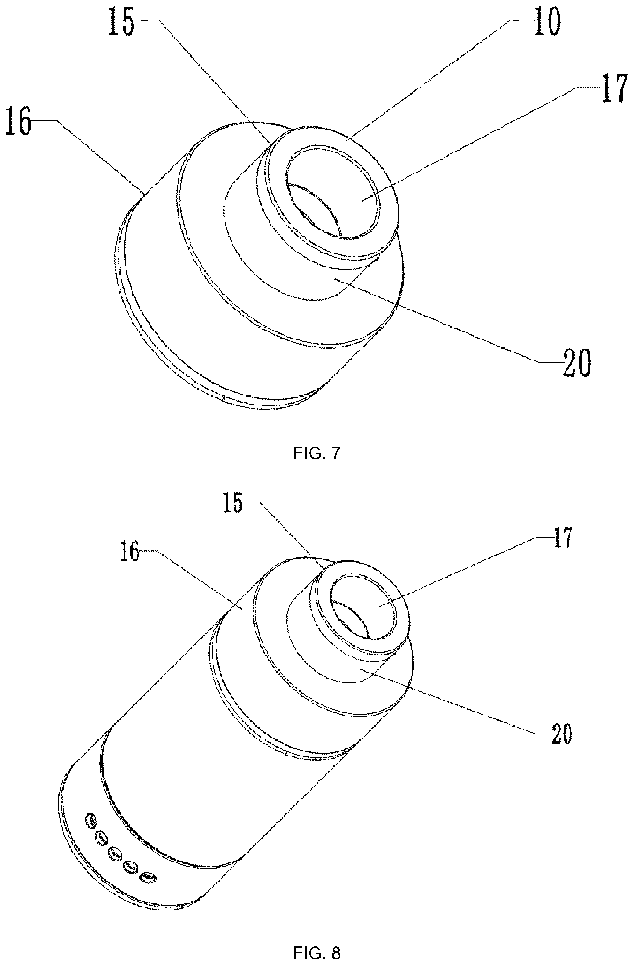

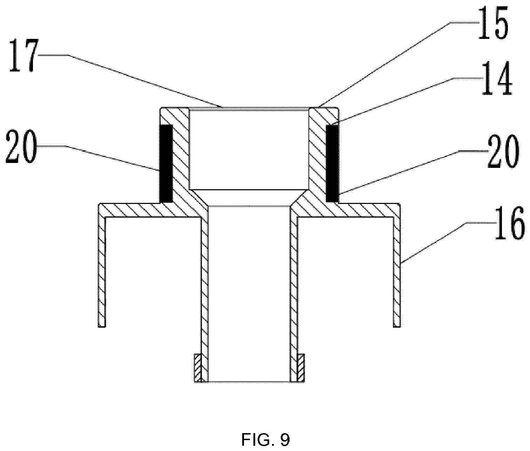

As shown in FIGs.7-9, in another embodiment, the hard substrate 10 has an approximately cylindrical shape and comprises two sections with different diameters, that is, an upper section 15 and a lower section 16. A through hole is defined inside the hard substrate 10 and extends through the upper section 15 and the lower section 16. The top end of the through hole is the smoke outlet 17. The diameter of the upper section 15 is relatively smaller, and that of the lower section 16 is relatively larger. A groove 14 in an annular shape is defined on the outside of the upper section 15 along the circumferential direction of the upper section 15. The soft substrate 20 is mounted on the upper section 15 along the circumferential direction of the upper section 15. Specifically, the soft substrate 20 has a sleeve shape and located in the groove 14. When the soft substrate 20 is mounted in the groove 14, the outer surface of the entire upper section 15 becomes a smooth curved surface.

In one embodiment, there is no concave portion 18 defined on the hard substrate 10. That is, between the top end 11 and the bottom end 12, there is no curved surface or planar surface defined inclining to the central axis of the cigarette mouthpiece. Furthermore, the shape of the hard substrate 10 is selected from one of a group of cylinder, frustum of a prism and frustum of a cone. Furthermore, a groove 14 is defined on the hard substrate 10, and the soft substrate 20 is mounted in the groove 14. Alternatively, there is no groove 14 defined on the hard substrate 10, and the soft substrate 20 is directly mounted on the hard substrate 10.

In one embodiment, the connection method between the soft substrate 20 and the hard substrate 10 includes, but is not limited to, adhesion and sleeving.

In one embodiment, a cartomizer is provided. The cartomizer comprises a main body 31 and a cigarette mouthpiece mounted on the top end of the main body 31. A liquid storage chamber is defined in the main body 31. The cigarette mouthpiece is any one of the cigarette mouthpieces described in the previous embodiments.

In one embodiment, an electronic cigarette is provided which comprises a cartomizer described in the previous embodiment.

In one embodiment, an electronic cigarette is provided which comprises a cigarette mouthpiece described in any one of the previous embodiments.

The cigarette mouthpiece provided in the present disclosure comprises a hard substrate 10 and a soft substrate 20 mounted on the hard substrate 10. Accordingly, the user's lips can obtain soft and comfortable tactility when in contact with the cigarette mouthpiece. At the same time, the use of the hard substrate 10 in cooperation with the soft substrate 20 ensures the reliability of the cigarette mouthpiece, reduces the risk of contamination and prolongs the service life.

## Claims

1. A cigarette mouthpiece for an electronic cigarette comprising a hard substrate (10) having a top end (11) and a bottom end (12), and a soft substrate (20), a through hole being defined inside the hard substrate (10) and extending through the opposite ends of the hard substrate (10), the through hole being configured as a smoke passage, an opening of the through hole located in the top end (11) of the through hole being configured as a smoke outlet (17), the soft substrate (20) being mounted on the outside of the hard substrate (10) and supported by the hard substrate (10), wherein a concave portion (18) is located between the top end (11) and the bottom end (12), a groove (14) is defined on the concave portion (18), and the soft substrate (20) is mounted in the groove (14), **characterized in that** both the number of the groove (14) and the soft substrate (20) are two, the two grooves (14) are respectively defined on the opposite sides of the hard substrate (10), and the two soft substrates (20) are respectively mounted in the corresponding grooves (14).

2. The cigarette mouthpiece according to claim 1, wherein the soft substrate (20) is a sheet, and the two ends of the soft substrate (20) are relatively thinner than the middle portion of the soft substrate (20).

3. The cigarette mouthpiece according to claim 1, wherein the soft substrate (20) is melted at a high temperature and then adhered to the groove (14).

4. The cigarette mouthpiece according to claim 1, wherein the soft substrate (20) is disposed around the hard substrate (10).

5. The cigarette mouthpiece according to claim 4, wherein the soft substrate (20) is mounted on the outside of the hard substrate (10) through the cooperation of a retaining protrusion (13) defined on the concave portion (18) and a retaining hole (21) defined on the soft substrate (20), alternatively, two retaining protrusions (13) are defined on the hard substrate (10) along the circumferential direction of the hard substrate (10), one retaining protrusion (13) is adjacent to the top end (11), the other retaining protrusion (13) is adjacent to the bottom end (12), and the soft substrate (20) is mounted between the two retaining protrusions (13).

6. The cigarette mouthpiece according to claim 1, wherein the hard substrate (10) comprises an upper section (15) and a lower section (16), a groove (14) is defined on the outside of the upper section (15) along the circumferential direction of the upper section (15), and the soft substrate (20) is mounted in the groove (14) along the circumferential direction of the upper section (15).

7. The cigarette mouthpiece according to any one of claims 1 to 6, wherein the hard substrate (10) is made of one of a group of hard plastic, stainless steel and glass.

8. The cigarette mouthpiece according to any one of claims 1to 6, wherein the soft substrate (20) is made of one of a group of silicon resin, latex, TPE, EVA and soft silicone rubber.

9. A cartomizer comprising a main body and a cigarette mouthpiece claimed in any one of claims 1 to 8, and the cigarette mouthpiece being mounted on the main body (31).

10. An electronic cigarette comprising a cigarette mouthpiece claimed in any one of claims 1 to 8.

## Patentansprüche

1. Zigarettenmundstück für eine elektronische Zigarette, umfassend ein hartes Substrat (10), das ein oberes Ende (11) und ein unteres Ende (12) aufweist, und ein weiches Substrat (20), wobei ein Durchgangsloch innerhalb des harten Substrats (10) definiert ist und die gegenüberliegenden Enden des harten Substrats (10) durchläuft, wobei das Durchgangsloch als ein Rauchdurchgang konfiguriert ist, wobei eine Öffnung des Durchgangslochs, die sich im oberen Ende (11) des Durchgangslochs befindet, als ein Rauchauslass (17) konfiguriert ist, wobei das weiche Substrat (20) auf der Außenseite des harten Substrats (10) angebracht und durch das harte Substrat (10) gestützt ist, wobei sich ein konkaver Abschnitt (18) zwischen dem oberen Ende (11) und dem unteren Ende (12) befindet, eine Nut (14) am konkaven Abschnitt (18) definiert ist und das weiche Substrat (20) in der Nut (14) angebracht ist, **dadurch gekennzeichnet, dass** die Anzahl sowohl der Nut (14) als auch des weichen Substrats (20) zwei beträgt, die zwei Nute (14) jeweils auf gegenüberliegenden Seiten des harten Substrats (10) definiert sind und die zwei weichen Substrate (20) jeweils in den entsprechenden Nuten (14) angebracht sind.

2. Zigarettenmundstück nach Anspruch 1, wobei das weiche Substrat (20) eine Folie ist und die zwei Enden des weichen Substrats (20) relativ dünner als der mittlere Abschnitt des weichen Substrats (20) sind.

3. Zigarettenmundstück nach Anspruch 1, wobei das weiche Substrat (20) auf einer hohen Temperatur geschmolzen und dann an die Nut (14) angehaftet wird.

4. Zigarettenmundstück nach Anspruch 1, wobei das weiche Substrat (20) um das harte Substrat (10) herum angeordnet ist.

5. Zigarettenmundstück nach Anspruch 4, wobei das weiche Substrat (20) auf der Außenseite des harten Substrats (10) durch die Zusammenwirkung eines Haltevorsprungs (13), der am konkaven Abschnitt (18) definiert ist, und eines Haltelochs (21), das am weichen Substrat (20) definiert ist, angebracht ist, wobei alternativ zwei Haltevorsprünge (13) auf dem harten Substrat (10) entlang der Umfangsrichtung des harten Substrats (10) definiert sind, ein Haltevorsprung (13) dem oberen Ende (11) benachbart ist, der andere Haltevorsprung (13) dem unteren Ende (12) benachbart ist und das weiche Substrat (20) zwischen den zwei Haltevorsprüngen (13) angebracht ist.

6. Zigarettenmundstück nach Anspruch 1, wobei das harte Substrat (10) einen oberen Teilabschnitt (15) und einen unteren Teilabschnitt (16) umfasst, eine Nut (14) auf der Außenseite des oberen Teilabschnitts (15) entlang der Umfangsrichtung des oberen Teilabschnitts (15) definiert ist und das weiche Substrat (20) in der Nut (14) entlang der Umfangsrichtung des oberen Teilabschnitts (15) angebracht ist.

7. Zigarettenmundstück nach einem der Ansprüche 1 bis 6, wobei das harte Substrat (10) aus einem einer Gruppe von hartem Kunststoff, Edelstahl und Glas hergestellt ist.

8. Zigarettenmundstück nach einem der Ansprüche 1 bis 6, wobei das weiche Substrat (20) aus einem einer Gruppe von Silikonharz, Latex, TPE, EVA und weichem Silikonkautschuk hergestellt ist.

9. Cartomizer, umfassend einen Hauptkörper und ein Zigarettenmundstück nach einem der Ansprüche 1 bis 8, und wobei das Zigarettenmundstück am Hauptkörper (31) angebracht ist.

10. Elektronische Zigarette, umfassend ein Zigarettenmundstück nach einem der Ansprüche 1 bis 8.

## Revendications

1. Embout buccal de cigarette pour cigarette électronique, comprenant un substrat dur (10) doté d'une extrémité supérieure (11) et d'une extrémité inférieure (12), et un substrat mou (20), un trou traversant étant défini à l'intérieur du substrat dur (10) et s'étendant à travers les extrémités opposées du substrat dur (10), le trou traversant étant conçu sous forme d'un passage à fumée, une ouverture du trou traversant située dans l'extrémité supérieure (11) du trou traversant étant conçue sous forme d'une sortie de fumée (17), le substrat mou (20) étant monté sur l'extérieur du substrat dur (10) et supporté par le substrat dur (10), une section concave (18) étant située entre l'extrémité supérieure (11) et l'extrémité inférieure (12), une gorge (14) étant définie sur la section concave (18), et le substrat mou (20) étant monté dans la gorge (14), **caractérisé en ce que** le nombre de gorges (14) et de substrat mou (20) est chaque fois de deux, les deux gorges (14) étant respectivement définies sur les faces opposées du substrat dur (10), et les deux substrats mous (20) étant respectivement montés dans les gorges correspondantes (14).

2. Embout buccal de cigarette selon la revendication 1, dans lequel le substrat mou (20) est une feuille, et les deux extrémités du substrat mou (20) sont relativement plus minces que la section médiane du substrat mou (20).

3. Embout buccal de cigarette selon la revendication 1, dans lequel le substrat mou (20) est fondu à haute température puis collé dans la gorge (14).

4. Embout buccal de cigarette selon la revendication 1, dans lequel le substrat mou (20) est disposé autour du substrat dur (10).

5. Embout buccal de cigarette selon la revendication 4, dans lequel le substrat mou (20) est monté sur l'extérieur du substrat dur (10) par la coopération d'une saillie de rétention (13) définie sur la section concave (18) et d'un trou de rétention (21) défini sur le substrat mou (20) alternativement, deux saillies de rétention (13) sont définies sur le substrat dur (10) dans le sens circonférentiel du substrat dur (10), une saillie de rétention (13) est adjacente à l'extrémité supérieure (11), l'autre saillie de rétention (13) est adjacente à l'extrémité inférieure (12), et le substrat mou (20) est monté entre les deux saillies de rétention (13).

6. Embout buccal de cigarette selon la revendication 1, dans lequel le substrat dur (10) comprend une section supérieure (15) et une section inférieure (16), une gorge (14) est définie du sur l'extérieur de la section supérieure (15) dans le sens circonférentiel de la section supérieure (15), et le substrat mou (20) est monté dans la gorge (14) dans le sens circonférentiel de la section supérieure (15).

7. Embout buccal de cigarette selon l'une quelconque des revendications 1 à 6, dans lequel le substrat dur (10) est composé d'un matériau d'un groupe composé du plastique dur, de l'acier inoxydable et du verre.

8. Embout buccal de cigarette selon l'une quelconque des revendications 1 à 6, dans lequel le substrat mou (20) est composé d'un matériau d'un groupe composé de la résine de silicone, du latex, du TPE, de l'EVA et du caoutchouc silicone mou.

9. Cartomiseur comprenant un corps principal et un embout buccal de cigarette selon l'une quelconque des revendications 1 à 8, l'embout buccal de cigarette étant monté sur le corps principal (31).

10. Cigarette électronique comprenant un embout buccal de cigarette selon l'une quelconque des revendications 1 à 8.
